(19) Europäisches Patentamt
European Patent Office
Office européen des brevets

(11) EP 1 047 719 B1

(12) **EUROPÄISCHE PATENTSCHRIFT**

(45) Veröffentlichungstag und Bekanntmachung des Hinweises auf die Patenterteilung:
**03.04.2002 Patentblatt 2002/14**

(21) Anmeldenummer: **98966389.3**

(22) Anmeldetag: **18.12.1998**

(51) Int Cl.[7]: **C08G 18/78**, C09D 175/04, C08G 18/79, C08G 18/80

(86) Internationale Anmeldenummer:
**PCT/EP98/08383**

(87) Internationale Veröffentlichungsnummer:
**WO 99/36455 (22.07.1999 Gazette 1999/29)**

# (54) MISCHUNGEN ENTHALTEND DIISOCYANATE MIT ALLOPHANATGRUPPEN ABGELEITET VON ALICYCLISCHEN ALKOHOLEN

MIXTURES CONTAINING DIISOCYANATES WITH ALLOPHANATE GROUPS DERIVED FROM ALICYCLIC ALCOHOLS

MELANGES COMPRENANT DES DIISOCYANATES PORTEURS DE GROUPES ALLOPHANATE DERIVES D'ALCOOLS ALICYCLIQUES

(84) Benannte Vertragsstaaten:
**BE DE ES FR GB IT NL SE**

(30) Priorität: **16.01.1998 DE 19801322**

(43) Veröffentlichungstag der Anmeldung:
**02.11.2000 Patentblatt 2000/44**

(73) Patentinhaber: **BASF AKTIENGESELLSCHAFT**
**67056 Ludwigshafen (DE)**

(72) Erfinder:
- **BRUCHMANN, Bernd**
**D-67251 Freinsheim (DE)**
- **RENZ, Hans**
**D-67149 Meckenheim (DE)**
- **MOHRHARDT, Günter**
**D-67346 Speyer (DE)**
- **BÖCK, Harald**
**D-67141 Neuhofen (DE)**

(56) Entgegenhaltungen:
**EP-A- 0 535 483**
**DE-A- 4 229 183**
**US-A- 5 290 902**
**US-A- 5 789 519**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 047 719 B1

Printed by Jouve, 75001 PARIS (FR)

## Beschreibung

Die vorliegende Erfindung betrifft Mischungen enthaltend Diisocyanate der allgemeinen Formel (I)

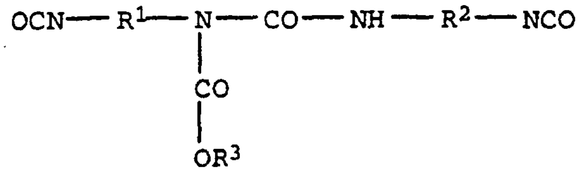

(I)

in der die Reste die folgende Bedeutung haben:

$R^1$, $R^2$: beide Reste ein Rest der Formel (II)

$$-CH_2-CH_2-CH_2-CH_2-CH_2-CH_2-$$

(Diisocyanate Ia) (II)

ein Rest der Formel (II) und der andere Rest ein Rest der Formel (III)

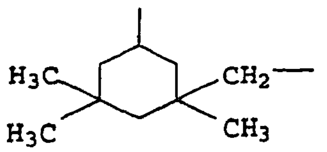

(III)

(Diisocyanate Ib)

$R^3$: ein 5 oder 6 gliedriger Cycloalkylrest, bei dem bis zu 3 Wasserstoffatome durch $C_1$- bis $C_4$-Alkylreste und ein oder zwei Methyleneinheiten durch ein Sauerstoffatom und/oder eine tertiäres Stickstoffatom, das zusätzlich einen $C_1$- bis $C_4$-Alkylrest trägt, substituiert sein können oder
ein $C_1$- bis $C_4$-Alkylrest, bei dem ein Wasserstoffatom substituiert ist durch einen 5 oder 6 gliedrigen Cycloalkylrest, bei dem bis zu 3 Wasserstoffatome durch $C_1$- bis $C_4$-Alkylreste und ein oder zwei. Methyleneinheiten durch ein Sauerstoffatom und/oder eine tertiäres Stickstoffatom, das zusätzlich einen $C_1$- bis $C_4$-Alkylrest trägt, substituiert sein können, einen Pyrrolidon- oder einen Morpholinrest, wobei bei den beiden letztgenannten Resten das N-Atom an den Alkylrest gebunden ist.

Weiterhin betrifft die Erfindung Isocyanat-Mischungen und 2-Komponenten-Beschichtungsmittel, die diese Isocyanate enthalten. Beschichtungsverfahren, bei denen diese 2-Komponenten-Beschichtungsmittel eingesetzt werden sowie die nach diesen Verfahren hergestellten beschichteten Gegenstände.

Vernetzende Polyisocyanate und Bindemittelkomponenten mit gegenüber Isocyanaten reaktiven Gruppen sind z.B. in Form von 2-K-Lakken allgemein bekannt (vgl. Kunststoff Handbuch, Band 7, Polyurethan, 2. Auflage, 1983, Carl Hanser Verlag München Wien, S. 540 bis 561). Diese 2-Komponenten-Beschichtungsmittel enthalten als Bindemittel beispielsweise ein polymeres Polyol und als Vernetzerkomponente (Härter) eine Verbindung mit mehreren freien Isocyanatgruppen.

Die Gebrauchseigenschaften von Lacken. deren polymere Bindemittel mit den Isocyanaten vernetzt wurden, sind gegenüber Systemen mit niedermolekularen Bindemitteln deutlich verbessert. Dies betrifft insbesondere Gebrauchseigenschaften wie

- Unempfindlichkeit gegenüber mechanischer Beanspruchung wie Zug, Dehnung, Schlägen oder Abrieb
- Resistenz gegenüber Feuchtigkeit (z.B. in Form von Wasserdampf) und verdünnten Chemikalien
- Beständigkeit gegenüber Umwelteinflüssen wie Temperaturschwankungen und UV-Strahlung

- hoher Glanz der beschichteten Oberflächen.

Es wird erwartet, daß die Härter nicht nur den ausgehärteten Lakken die vorgenannten Gebrauchseigenschaften verleihen, sondern auch die verarbeitungstechnischen Eigenschaften der Lacke vor deren Auftrag verbessert oder zumindest möglichst wenig beeinträchtigt.

Damit die Lacke problemlos mit üblichen Verfahren, z.B. durch Aufsprühen auf die zu beschichtende Oberfläche. aufgetragen werden können, sollen die Lacke eine begrenzte Viskosität aufweisen. Lacke auf Basis von 2-Komponenten-Beschichtungsmitteln enthalten deshalb üblicherweise Lösungsmittel. Der hohe Lösungsmittelgehalt dieser Lacke bereitet jedoch Probleme, da die Verarbeiter der Lacke technisch aufwendige Maßnahmen ergreifen müssen, um zu vermeiden, daß die Lösungsmittel, die beim Auftrag und Trocknen der Lacke freigesetzt werden, in die Atmosphäre gelangen. Es wurden deshalb Härter gesucht, die die Viskosität der Bindemittel-haltigen Komponente möglichst wenig erhöhen oder sogar besser noch erniedrigen. Selbstverständlich dürfen diese Härter selbst bei Raumtemperatur keine nennenswerte Flüchtigkeit aufweisen, wie dies bei handelsüblichen monomeren Isocyanaten wie Hexamethylendiisocyanat oder Isophorondiisocyanat der Fall ist.

Weiterhin sollen die 2-Komponenten-Beschichtungsmittel nach der Applikation möglichst rasch aushärten, so daß die beschichteten Gegenstände nach dem Auftrag rasch weiterverarbeitet oder benutzt werden können.

Polyisocyanate, die Allophanat- und Biuretgruppen enthalten, sind beispielsweise aus der EP-A-496208, 524501 und 566037 bekannt und werden dort für den Einsatz als Härter in 2-Komponenten-Beschichtungssystemen empfohlen. Als Aufbaukomponenten kommen aliphatische Monoalkohole und alle üblichen Isocyanate in Betracht. Die Aufbaukomponenten aus der Gruppe der cyclischen Monoalkohole bzw. Hexamethylendiisocyanat und Isophorondiisocyanat finden sich in diesen Schriften lediglich im Rahmen von Aufzählungen der geeigneten Isocyanat- und Alkoholkomponenten, die in Form von Listen zusammengefaßt sind. Wie aus dem experimentellen Teil dieser Schriften hervorgeht, liegt der Schwerpunkt auf Polyisocyanaten mit Allophanat- und Biuretgruppen, die sich von nicht-cyclischen Alkoholen ableiten.

Die aus diesen Alkoholen abgeleiteten Allophanate weisen insbesondere den Nachteil auf, daß die damit hergestellten 2-K-Beschichtungssysteme relativ langsam aushärten und auch nach vollständiger Aushärtung eine für manche Anwendungszwecke noch zu geringe Oberflächenhärte aufweisen.

Aus der DE-A-4229183 sind Urethangruppen- und Isocyanuratgruppenhaltige Polyisocyanate mit niedriger Viskosität bekannt. Zu deren Herstellung werden z.B. cycloaliphatische Alkohole wie Cyclohexanol mit einem Isocyanurat des Hexamethylendiisocyanats zum entsprechenden Urethan umgesetzt.

Die Eigenschaften der dort offenbarten Härter befriedigen zwar meist die üblichen Anforderungen, die bezüglich der verarbeitungstechnischen Eigenschaften der unausgehärteten flüssigen Lacksysteme, die diese Härter enthalten, und der Gebrauchseigenschaften der mit den Lacken hergestellten Beschichtungen gestellt werden. Bezüglich der Viskosität der Lacksysteme, deren Aushärtungsgeschwindigkeit sowie der Härte der damit hergestellten Beschichtungen erscheinen die Härter allerdings noch verbesserungsbedürftig.

Demgemäß wurden die eingangs definierten Verbindungen der Formel (I), Mischungen und 2-Komponenten-Beschichtungsmittel, die diese Verbindungen enthalten sowie Gegenstände, die mit diesen 2-Komponenten-Beschichtungsmitteln beschichtet sind, gefunden.

Die erfindungsgemäßen Diisocyanate der Formel (I) leiten sich bevorzugt von Alkoholen wie Cyclohexanol, Cyclohexanmethanol, Cyclopentanol, Cyclopentanmethanol, 3,3,5-Trimethylcyclohexanol, Menthol, Norborneol, N-Methyl-4-hydroxypiperidin, 4-(2-Hydroxyethyl) -morpholin oder 4-(2-Hydroxyethyl)-pyrrolidon ab.

Im allgemeinen lassen sich diese Diisocyanate herstellen, indem man

(i) Isophorondiisocyanat (IPDI), Hexamethylendiisocyanat (HDI), oder eine Mischung dieser Isocyanate mit einem

- 5 oder 6 gliedrigen cycloaliphatischen Alkohol, bei dem bis zu 3 an ein C-Atom gebundene Wasserstoffatome durch $C_1$- bis $C_4$-Alkylreste und ein oder zwei Methyleneinheiten durch ein Sauerstoffatom und/oder ein tertiäres Stickstoffatom, das zusätzlich eine $C_1$- bis $C_4$-Alkylrest trägt, substituiert sein können oder

- einem $C_1$- bis $C_4$-Alkylalkohol, bei dem ein an ein C-Atom gebundenes Wasserstoffatom substituiert ist durch einen 5 oder 6 gliedrigen Cycloalkylrest, bei dem bis zu 3 Wasserstoffatome durch $C_1$- bis $C_4$-Alkylreste und ein oder zwei Methyleneinheiten durch ein Sauerstoffatom und/oder ein tertiäres Stickstoffatom, das zusätzlich eine $C_1$- bis $C_4$-Alkylrest trägt, substituiert sein können, einen Pyrrolidon- oder Morpholinrest, wobei bei den beiden letztgenannten Resten das N-Atom an den Alkylrest des Alkohols gebunden ist,

wobei das Molverhältnis der genannten Isocyanate zu dem genannten Monoalkohol 1.5:1 bis 20:1 beträgt, in Gegenwart eines Katalysators umsetzt.

(ii) den Katalysator desaktiviert und

(iii) gegebenenfalls nicht umgesetztes Isocyanat entfernt.

Das molare Mischungsverhältnis der HDI/IPDI-Mischungen liegt bevorzugt bei 0,1:1 bis 10:1.

Die Umsetzung kann beispielsweise auf die Weise vorgenommen werden, wie sie in der EP-A-524501 beschrieben ist.

Als Katalysatoren dienen beispielsweise quartäre Ammoniumsalze, z.B. N,N,N-Trimethyl-N-(2-hydroxypropyl)ammonium-2-ethylhexanoat.

Die Umsetzung wird im allgemeinen bei Temperaturen von 50 bis 150°C durchgeführt.

Die Reaktion wird beendet, indem man die Reaktionsmischung abkühlt und den Katalysator entfernt, thermisch zersetzt oder ein geeignetes Mittel zusetzt, das den Katalysator desaktiviert. Hierzu sind beispielsweise Säuren wie p-Toluolsulfonsäuren, Dibutylphosphat oder Di-(2-Ethylhexyl)phosphat geeignet.

Nach Beendigung der Reaktion destilliert man im allgemeinen unumgesetztes Hexamethylendiisocyanat oder Isophorondiisocyanat, bevorzugt bis zu einem Gehalt von weniger als 0,5 Gew.-%, ab.

Das Umsetzungsprodukt, das im wesentlichen frei von Isophorondiisocyanat oder Hexamethylendiisocyanat ist, weist im allgemeinen eine Viskosität von 100 bis 10000, bevorzugt von 150 bis 6000 mPas, gemessen nach ISO 3219, auf.

Nach diesem Verfahren erhält man im allgemeinen Mischungen, in denen neben den Diisocyanaten (Ia), (Ib) und/oder (Ic),

- Urethane der Formel (IV)

$$OCN\text{-}R^1\text{-}NH\text{-}CO\text{-}OR^3 \qquad (IV)$$

in der die Reste $R^1$ und $R^3$ die folgende Bedeutung haben können:

$R^1$ ein Rest der Formel (II) oder (III),

$R^3$ die in Anspruch 1 angegebene Bedeutung,

- Diisocyanate der Formel (V),

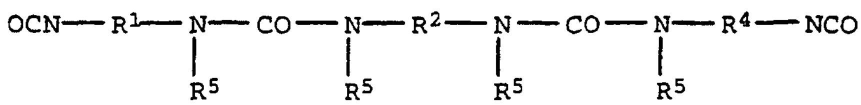

(V)

in der die Reste $R^1$, $R^2$, $R^4$ und $R^5$ die folgende Bedeutung haben können:

$R^1$, $R^2$, $R^4$ die für $R^1$ Formel (I) angegebene Bedeutung,

$R^5$ 2 der insgesamt 4 Reste Wasserstoff und die beiden anderen Reste ein Rest der Formel VI

$$\text{-}CO\text{-}O\text{-}R^3 \qquad (VI)$$

sind, wobei die Reste $R^5$ mit der gleichen Bedeutung durch die Einheit $R^2$ getrennt sind und

- Isocyanurate, aufgebaut aus 3 Molekülen, ausgewählt aus der Gruppe Isophorondiisocyanat oder Hexamethylendiisocyanat, enthalten sein können.

In diesen Mischungen beträgt im allgemeinen

- der Anteil der Summe der Diisocyanate (Ia), (Ib) und (Ic) 5 bis 100 Gew.-%
- der Anteil der Urethane der Formel (IV) 0 bis 20 Gew.-%,
- der Anteil der Diisocyanate der Formel (V) 0 bis 30 Gew.-% und
- der Anteil der Isocyanurate, aufgebaut aus je 3 Molekülen, ausgewählt aus der Gruppe Isophorondiisocyanat oder Hexamethylendiisocyanat 0 bis 65 Gew.-%.

Vorzugsweise beträgt das Gew.-Verhältnis von Diisocyanaten (I) zu Monoisocyanuraten (VII) 10:1 bis 1:10.

Die Summe der Anteile der Diisocyanate (Ia), (Ib), (Ic), (V), des Urethans (IV) sowie des Isocyanurates (VII) macht bevorzugt 10 bis 100 Gew.-% aus.

Aus diesen Mischungen lassen sich, falls gewünscht, die erfindungsgemäßen Diisocyanate einfach durch bekannte Trennmethoden wie die Gelpermeationschromatographie isolieren. Dies ist jedoch im allgemeinen nicht erforderlich, sofern diese Diisocyanate (Ia), (Ib), (Ic), in Form der vorgenannten Mischungen als Vernetzer in 2-Komponenten-Polyurethanbeschichtungsmitteln eingesetzt werden.

Die Diisocyanate (Ia), (Ib), (Ic) und die vorgenannten Mischungen, die diese Diisocyanate enthalten, eignen sich insbesondere als B-Komponente bei der Herstellung von 2-Komponenten-Beschichtungsmassen, die als A-Komponente eine Verbindung, die mit Polyisocyanat reagierende Gruppen trägt, bevorzugt ein hydroxyfunktionelles Polymer (A), enthalten.

Bei den hydroxyfunktionellen Polymeren (A) handelt es sich z.B. um Polymere mit einem Gehalt an Hydroxylgruppen von 0,1 bis 20, vorzugsweise 0,5 bis 10 Gew.-%. Das zahlenmittlere Molekulargewicht $M_n$ der Polymeren beträgt vorzugsweise 1000 bis 100 000, besonders bevorzugt 2000 bis 10 000. Bei den Polymeren handelt es sich bevorzugt um solche, welche zu mehr als 50 Gew.-% aus $C_1$-$C_{20}$-Alkyl(meth)acrylat, Vinylaromaten mit bis zu 20 C-Atomen, Vinylestern von bis zu 20 C-Atomen enthaltenden Carbonsäuren, Vinylhalogeniden, nicht aromatischen Kohlenwasserstoffen mit 4 bis 8 C-Atomen und 1 oder 2 Doppelbindungen, ungesättigten Nitrilen und deren Mischungen bestehen. Besonders bevorzugt sind die Polymeren, die zu mehr als 60 Gew.-% aus $C_1$-$C_{10}$-Alkyl-(meth)-acrylaten, Styrol oder deren Mischungen bestehen.

Darüber hinaus enthalten die Polymeren (A) hydroxyfunktionelle Monomere entsprechend dem obigen Hydroxylgruppengehalt und gegebenenfalls weitere Monomere, z.B. ethylenisch ungesättigte Säuren, insbesondere Carbonsäuren, Säureanhydride oder Säureamide.

Weitere Polymere (A) sind z.B. Polyesterole, wie sie durch Kondensation von Polycarbonsäuren, insbesondere Dicarbonsäuren mit Polyolen, insbesondere Diolen erhältlich sind.

Weiterhin sind als Polymere (A) auch Polyetherole geeignet, die durch Addition von Ethylenoxid, Propylenoxid oder Butylenoxid an H-aktive Komponenten hergestellt werden. Ebenso sind Polykondensate aus Butandiol geeignet.

Bei den Polymeren (A) kann es sich natürlich auch um Verbindungen mit primären oder sekundären Aminogruppen handeln.

Genannt seien z.B. sogenannte Jeffamine, d.h. mit Aminogruppen terminierte Polyetherole oder Oxazolidine.

Neben den vorstehend aufgeführten A- und B-Komponenten können in den 2-Komponenten-Beschichtungsmittel weiterhin sonstige Polyisocyanate und Verbindungen mit gegenüber Polyisocyanaten reaktionsfähigen Gruppen enthalten sein, wie sie üblicherweise in Zweikomponenten-Beschichtungsmassen vorhanden sind.

Bevorzugt beträgt das molare Verhältnis, gebildet aus der Summe der Isocyanatgruppen in den B-Komponenten gegenüber der Summe der mit Isocyanatgruppen reaktiven Gruppen der Komponente (A) 0,6:1 bis 1,4:1, bevorzugt 0,7:1 bis 1,3:1. ganz besonders bevorzugt 1:1.

Die erfindungsgemäßen Beschichtungsmassen können weiterhin noch organische Lösemittel, z.B. Xylol, Butylacetat, Methylisobutylketon, Methoxypropylacetat, N-Methylpyrrolidon enthalten. Mit Lösemittel wird die zur Verarbeitung, d.h. zum Auftragen auf Substrate, gewünschte niedrige Viskosität der Beschichtungsmasse eingestellt.

Die Beschichtungsmassen können natürlich weitere, in der Beschichtungstechnologie übliche Zusatzstoffe, z.B. Pigmente, Füllstoffe, Verlaufshilfsmittel etc. enthalten.

Sie können weiterhin Katalysatoren für die Urethanbildung, z.B. Dibutylzinndilaurat, enthalten.

Die Herstellung der Zweikomponenten-Polyurethanbeschichtungsmittel kann in bekannter Weise erfolgen. Gewöhnlich werden die A- und die B-Komponente vor dem Auftrag der Beschichtungsmittel auf ein Substrat gemischt. Die Vermischung erfolgt üblicherweise 0 bis 8 h vor dem Auftrag. Mit Lösungsmittel kann die gewünschte Viskosität eingestellt werden.

Die Polyurethanbeschichtungsmittel können in üblicher Weise durch Spritzen, Gießen, Walzen, Streichen, Rakeln etc. z.B. innerhalb von 12h nach ihrer Herstellung auf Substrate flächig aufgebracht werden.

Die Beschichtungsmittel eignen sich insbesondere für Werkstücke mit Oberflächen aus Metall, Kunststoff, Holz, Holzwerkstoffen oder Glas.

Die gemäß diesen Verfahren beschichteten Gegenstände weisen eine Oberfläche mit sehr guten mechanische Eigenschaften, insbesondere einer hohe Härte, Flexibilität und Chemikalienbeständigkeit auf. Diese Eigenschaften werden mit den erfindungsgemäßen Beschichtungsmitteln schon nach besonders kurzer Härtungszeit erreicht.

Experimenteller Teil

A. Herstellung der Urethan- und Allophanatgruppen enthaltenden Polyisocyanate

A.1 Herstellung der Urethan- und Allophanatgruppen enthaltenden Polyisocyanate aus HDI und Monoalkoholen, sowie Vergleichsbeispiele

Hexamethylendiisocyanat (HDI) wurde unter Stickstoffbedeckung vorgelegt und die in Tabelle 1 genannte Menge an OH-Komponente zugesetzt. Man erwärmte die Mischung auf 80°C, gab 200 Gew. ppm (bezogen auf Diisocyanat) des Katalysators N,N,N-Trimethyl-N-(2-hydroxypropyl)ammonium-2-ethylhexanoat zu, ließ bei dieser Temperatur reagieren und stoppte die Reaktion bei dem in Tabelle 1 genannten NCO-Gehalt der Mischung durch Zugabe von 250 Gew. ppm (bezogen auf Diisocyanat) Di-(2-Ethylhexyl)phosphat. Das Reaktionsgemisch wurde anschließend zur Entfernung vom monomeren HDI im Dünnschichtverdampfer bei 165°C Öltemperatur und 2,5 mbar destilliert. Der HDI-Restmonomergehalt lag danach unter 0,2 Gew.%.

Daten zu den Endprodukten stehen in Tabelle 1.

A.2 Herstellung eines Urethan- und Allophanatgruppen enthaltenden Polyisocyanates aus IPDI und Cyclohexanol

6 mol IPDI wurden unter Stickstoffbedeckung vorgelegt und 1,2 mol (20 mol%) Cyclohexanol zugesetzt. Man erwärmte die Mischung auf 70°C, gab 800 Gew. ppm (bezogen auf Diisocyanat) des in A.1 genannten Katalysators zu, ließ bei dieser Temperatur reagieren und stoppte die Reaktion bei einem NCO-Gehalt der Mischung von 27 Gew. % durch Zugabe von 1000 Gew.ppm (bezogen auf Diisocyanat) Di-(2-Ethylhexyl)phosphat. Das Reaktionsgemisch wurde anschließend zur Entfernung von monomerem IPDI im Dünnschichtverdampfer bei 165°C Öltemperatur und 2,5 mbar destilliert.

Das feste Endprodukt hatte einen NCO-Gehalt von 15,4 % und einen Erweichungspunkt bei 43°C.

A.3 Herstellung eines Urethan- und Allophanatgruppen enthaltenden Polyisocyanates aus einer IPDI-HDI-Mischung und Cyclohexanol

Eine Mischung aus 4 mol HDI und 1,5 mol IPDI wurde unter Stickstoffbedeckung vorgelegt und mit 1,1 mol (20 mol%) Cyclohexanol versetzt. Man erwärmte die Mischung auf 80°C, gab 650 Gew. ppm (bezogen auf Diisocyanat) N,N,N-Trimethyl-N-(2-hydroxypropyl)ammonium-2-ethylhexanoat zu, ließ bei dieser Temperatur reagieren und stoppte die Reaktion bei einem NCO-Gehalt der Mischung von 31 Gew.% durch Zugabe von 800 Gew ppm (bezogen auf Diisocyanat) Di-(2-Ethylhexyl)-phosphat. Das Reaktionsgemisch wurde anschließend zur Entfernung vom monomerem IPDI im Dünnschichtverdampfer bei 165°C Öltemperatur und 2,5 mbar destilliert.

Das Endprodukt hatte einen NCO-Gehalt von 17,1 % und eine Viskosität bei 25°C von 4310 mPas.

B. Herstellung und Prüfung von Klarlacken aus den erfindungsgemäßen Polyisocyanaten

Die erfindungsgemäßen Polyisocyanate wurden exemplarisch mit einem hydroxifunktionellen Acrylatharz (Lumitol® H 136, BASF) sowie verschiedenen Polyisocyanathärtern (Vergleichslack 1: Basonat® HI 100, BASF, Vergleichslack 12: Allophanat auf Basis HDI mit 20 mol% Ethylhexanol) entsprechend den stöchiometrischen OH/NCO-Verhältnissen gemischt und bei Bedarf zur Beschleunigung der Aushärtung mit Dibutylzinndilaurat (DBTL, Merck) katalysiert. Die Einstellung auf eine Applikationsviskosität von 20 s (DIN 53 211 Becher 4 mm Auslaufdüse) erfolgte mit Butylacetat. Die Verarbeitungszeiten der Lacke wurden anhand der Katalysatordosierung so eingestellt, daß Trocknungszeiten von ca. 1 h erreicht wurden. Mit einem Filmziehrahmen wurden auf Glasplatten Beschichtungen mit einer Naßfilmdicke von 200 µm aufgetragen. Die so erhaltenen Klarlacke wurden 7 Tage unter Normklima gehärtet. Die Festgehalte wurden nach DIN V 53 216 1. Teil bestimmt.

Die mit den erfindungsgemäßen Vernetzern hergestellten Lacke weisen gegenüber einem Standardisocyanat (Lack-Nr.1 Vergl.) verbesserte Lackfestgehalte, bzw. einen geringeren Anteil an flüchtigen organischen Bestandteilen (VOC) auf. Das bedeutet, daß man, um Lacke mit gleicher Viskosität zu erhalten, im Fall der mit den erfindungsgemäßen Isocyanaten hergestellten Lacke weniger Lösungsmittel benötigt.

Gegenüber einem aus nicht-cycloaliphatischen Alkoholen aufgebauten Allophanat weisen die erfindungsgemäßen Lacke eine deutlich höhere Anfangs- und Endhärte auf (vgl. Tab. 4, Lack-Nr. 12 (Vergleichsvers.) mit den

erfindungsgemäßen Lakken). Bei Verwendung von N-Alkyl-Hydroxypiperidin erübrigt sich zudem der Zusatz eines Katalysators zur Aushärtung der Beschichtungsmittel.

Alle Lacke zeigen darüber hinaus eine gute Flexibilität, Haftung sowie eine gute Kratzbeständigkeit.

Tabelle 1:

| Reaktionsprodukte aus HDI und Monoalkoholen | | | | | |
|---|---|---|---|---|---|
| Versuch Nr. | Monoalkohol | Menge bez. Isocyanat (mol %) | NCO-Gehalt der Mischung (Gew.%) | NCO-Gehalt nach Destillation (Gew.%) | Viskosität bei 25°C (mPas) |
| 1 | Cyclohexanol | 5 | 40,2 | 20,7 | 1220 |
| 2 | Cyclohexanol | 10 | 36,9 | 19,5 | 1120 |
| 3 | Cyclohexanol | 15 | 35,7 | 18,3 | 890 |
| 4 | Cyclohexanol | 20 | 34,1 | 17,4 | 770 |
| 5 | Cyclopentanol | 5 | 40,1 | 21,0 | 980 |
| 6 | Cyclopentanol | 10 | 38,2 | 19,8 | 660 |
| 7 | Cyclopentanol | 15 | 36,8 | 18,3 | 610 |
| 8 | Cyclopentanol | 20 | 34,6 | 17,1 | 650 |
| 9 | Cyclohexan-methanol | 20 | 35,8 | 16,0 | 360 |
| 10 | Cyclopentan-methanol | 20 | 35,0 | 18,6 | 560 |
| 11 | 3,3,5-Trimethylcyclohexan | 10 | 36,2 | 18,3 | 1830 |
| 12 | 3,3,5-Trimethylcyclohexan | 20 | 32,2 | 15,9 | 1620 |
| 13 | Menthol | 20 | 34,6 | 14,5 | 1330 |
| 14 | Norborneol | 10 | 35,8 | 19,0 | 1810 |
| 15 = | Norborneol | 20 | 33,0 | 16,9 | 1320 |
| 16 | N-Methyl-4-hydroxypiperidin | 10 | 36,0 | 12,9 | 1320 |
| 17 | N-Methyl-4-hydroxypiperidin | 20 | 29,0 | 10,0 | 3340 |
| 18 | 4-(2-Hydroxyethyl)-morpholin | 5 | 39,1 | 18,3 | 4550 |
| 19 | 4-(2-Hydroxyethyl)-morpholin | 20 | 28,1 | 15,3 | 3580 |
| 20 | N-(2-Hydroxyethyl)-2-pyrrolidon | 20 | 32,3 | 16,2 | 2020 |
| 21 (Vergleich) | n-Butanol | 20 | 33,5 | 18,3 | 273 |
| 22 (Vergleich) | 2-Ethylhexanol | 20 | 31,2 | 16,5 | 343 |

Tabelle 2:

| Verbesserte VOC-Werte gegenüber Stand der Technik | | | | | | | |
|---|---|---|---|---|---|---|---|
| Lackzusammensetzung | | | | | | | |
| Lack | 1(Vgl.) | 2 | 3 | 4 | 5 | 6 | 7 |

Tabelle 2: (fortgesetzt)

| Verbesserte VOC-Werte gegenüber Stand der Technik | | | | | | | |
|---|---|---|---|---|---|---|---|
| Lackzusammensetzung | | | | | | | |
| Polyisocyanat (Tab.1) | HI 100 | 5 | 6 | 7 | 1 | 2 | 3 |
| Alkohol | - | Cyclopentanol | | | Cyclohexanol | | |
| | | | | | | | |
| Hydroxyacrylat | H 136 | H 136 | H 136 | H 136 | H 136 | H 136 | H 136 |
| % DBTL f.a.f.[1)] | 0,05 | 0,1 | 0,1 | 0,1 | 0,1 | 0,1 | 0,1 |
| Lackhärte | | | | | | | |
| Pendeldämpfung nach König [Schwingungen] | | | | | | | |
| nach 5h | 20 | 16 | 17 | 17 | 25 | 20 | 20 |
| 7d RT+15h 60°C | 143 | 139 | 142 | 144 | 141 | 140 | 141 |
| Flüchtige organische Bestandteile | | | | | | | |
| VOC g/l | 512 | 498 | 485 | 485 | 505 | 496 | 486 |

Tabelle 3:

| Verbesserte VOC-Werte gegenüber Stand der Technik | | | | | |
|---|---|---|---|---|---|
| Lackzusammensetzung | | | | | |
| Lack | 1(Vgl.) | 8 | 9 | 10 | |
| Polyisocyanat (Tab.1) | HI 100 | 11 | 14 | 16 | |
| Alkohol | - | 3,3,5-Trimethylcyclohexan | Norborneol | N-Methyl-4-hydroxy-piperidin | |
| | | | | | |
| Hydroxyacrylat | H 136 | H 136 | H 136 | H 136 | |
| % DBTL f.a.f.[1)] | 0,05 | 0,1 | 0,1 | - | |
| Lackhärte | | | | | |
| Pendeldämpfung nach König [Schwingungen] | | | | | |
| nach 5h | 20 | 13 | 13 | 111 | |
| 7d RT+15h 60°C | 143 | 144 | 140 | 130 | |
| Flüchtige organische Bestandteile | | | | | |
| VOC g/l | 512 | 495 | 492 | 477 | |

1) fest auf fest

Tabelle 4:

| Verbesserte Trocknungszeiten gegenüber niederviskosen Produkten nach Stand der Technik (vergleichbare VOC-Werte) | | | | |
|---|---|---|---|---|
| Lackzusammensetzung | | | | |
| Lack | 12(Vgl.) | 13 | 14 | 15 |
| Polyisocyanat (Tab.1) | 22 | 4 | 19 | 17 |
| Alkohol | 2-Ethylhexanol | Cyclohexanol | N-(2-Hydroxyethyl) morpholin | N-Methyl-4-hydroxypiperidin |

Tabelle 4: (fortgesetzt)

| | | | | |
|---|---|---|---|---|
| Verbesserte Trocknungszeiten gegenüber niederviskosen Produkten nach Stand der Technik (vergleichbare VOC-Werte) | | | | |
| Lackzusammensetzung | | | | |
| | | | | |
| Hydroxyacrylat | H 136 | H 136 | H 136 | H 136 |
| % DBTL f.a.f. | 0,1 | 0,1 | 0,1 | - |
| Lackhärte | | | | |
| Pendeldämpfung nach König [Schwingungen] | | | | |
| nach5h | 10 | 18 | 25 | 112 |
| 7d RT+15h 60°C | 117 | 136 | 135 | 135 |
| Flüchtige organische Bestandteile | | | | |
| VOC [g/l] | 477 | 476 | 494 | 475 |
| Lumitol H 136: Festgehalt = 70 %, OHZ = 135<br>Basonat HI 100: HDI-Polyisocyanat, Viskosität ca. 3000 mPas, Festgehalt = 100 %, NCO = 22 % | | | | |

**Patentansprüche**

**1.** Mischungen enthaltend

- Diisocyanate der Formel (I),

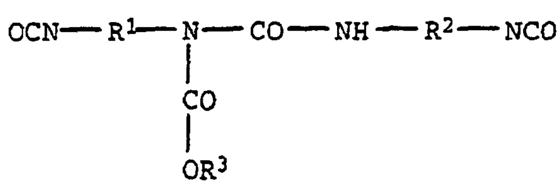

(I)

in der die Reste die folgende Bedeutung haben:

$R^1$, $R^2$: beide Reste ein Rest der Formel (II)

$$-CH_2-CH_2-CH_2-CH_2-CH_2-CH_2-$$

(Diisocyanate Ia) (II)

ein Rest der Formel (II) und der andere Rest ein Rest der Formel (III)

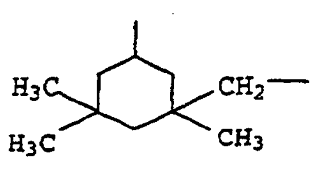

(III)

(Diisocyanate Ib)

$R^3$: - ein 5 oder 6 gliedriger Cycloalkylrest, bei dem bis zu 3 Wasserstoffatome durch $C_1$- bis $C_4$-Alkylreste und ein oder zwei Methyleneinheiten durch ein Sauerstoffatom und/oder eine tertiäres

Stickstoffatom, das zusätzlich einen $C_1$- bis $C_4$-Alkylrest trägt, substituiert sein können oder ein $C_1$- bis $C_4$-Alkylrest, bei dem ein Wasserstoffatom substituiert ist durch einen 5 oder 6 gliedrigen Cyclo-alkylrest, bei dem bis zu 3 Wasserstoffatome durch $C_1$-bis $C_4$-Alkylreste und ein oder zwei Methyleneinheiten durch ein Sauerstoffatom und/oder ein tertiäres Stickstoffatom, das zusätzlich einen $C_1$- bis $C_4$-Alkylrest trägt, substituiert sein können, einen Pyrrolidon- oder einen Morpholinrest, wobei bei den beiden letztgenannten Resten das N-Atom an den Alkylrest gebunden ist

- Urethane der Formel (IV)

$$OCN\text{-}R^1\text{-}NH\text{-}CO\text{-}OR^3 \quad (IV)$$

in der die Reste $R^1$ und $R^3$ die folgende Bedeutung haben können:

$R^1$ ein Rest der Formel (II) oder (III)
$R^3$ die in Anspruch 1 angegegebene Bedeutung,

- Diisocyanate der Formel (V),

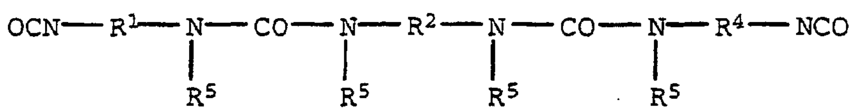

(V)

in der die Reste $R^1$, $R^2$, $R^4$ und $R^5$ die folgende Bedeutung haben können:

$R^1$, $R^2$, $R^4$ die für R1 in Formel (I) angegebene Bedeutung,

$R^5$ 2 der insgesamt 4 Reste Wasserstoff und die beiden anderen Reste ein Rest der Formel VI

$$\text{-}CO\text{-}O\text{-}R^3 \quad (VI)$$

sind, wobei die Reste $R^5$ mit der gleichen Bedeutung durch die Einheit $R^2$ getrennt sind und

- Isocyanurate, aufgebaut aus 3 Molekülen, ausgewählt aus der Gruppe Isophorondiisocyanat oder Hexamethylendiisocyanat (Monoisocyanurate VII),

wobei das Gew.-Verhältnis von Diisocyanaten (I) zu Monoisocyanuraten (VII) 10:1 bis 1:10 beträgt.

2. Mischungen enthalted Diisocyanate nach Anspruch 1, bei denen der Rest $R^3$ von einem Alkohol, ausgewählt aus der Gruppe Cyclohexanol, Cyclohexanmethanol, Cyclopentanol, Cyclopentanmethanol, 3,3,5-Trimethylcyclohexanol, Menthol, Norborneol, N-Methyl-4-hydroxypiperidin, 4-(2-Hydroxyethyl)-morpholin oder 4-(2-Hydroxyethyl)-pyrrolidon abgeleitet ist.

3. Mischungen nach Anspruch 1 oder 2, wobei der Anteil an Isophorondiisocyanat oder Hexamethylendiisocyanat weniger als 0,5 Gew.-% beträgt.

4. Mischungen nach den Ansprüchen 1 bis 3, wobei die Summe der Anteile der Diisocyanate (Ia), (Ib), (Ic), (V), des Urethans (IV) sowie des Isocyanurates (VII) 10 bis 100 Gew.-% ausmacht.

5. Verfahren zur Herstellung von Mischungen nach Anspruch 1 bis 4, **dadurch gekennzeichnet, daß** man

(i) Isophorondiisocyanat, Hexamethylendiisocyanat, oder eine Mischung dieser Isocyanate mit einem

- 5 oder 6 gliedrigen cycloaliphatischen Alkohol, bei dem bis zu 3 an ein C-Atom gebundene Wasserstoffatome durch $C_1$- bis $C_4$-Alkylreste und ein oder zwei Methyleneinheiten durch ein Sauerstoffatom und/oder ein tertiäres Stickstoffatom, das zusätzlich einen $C_1$- bis $C_4$-Alkylrest trägt, substituiert sein können oder

- $C_1$- bis $C_4$-Alkylalkohol, bei dem ein an ein C-Atom gebundenes Wasserstoffatom substituiert ist durch einen 5 oder 6 gliedrigen Cycloalkylrest, bei dem bis zu 3 Wasserstoffatome durch $C_1$- bis $C_4$-Alkylreste und ein oder zwei Methyleneinheiten durch ein Sauerstoffatom und/oder ein tertiäres Stickstoffatom, das zusätzlich einen $C_1$- bis $C_4$-Alkylrest trägt, substituiert sein können, einen Pyrrolidon- oder Morpholinrest,

wobei die beiden letztgenannten Reste über das N-Atom an den Alkylrest des Alkohols gebunden sind, wobei das Molverhältnis der genannten Isocyanate zu dem genannten Monoalkohol 1,5:1 bis 20:1 beträgt, in Gegenwart eines Katalysators umsetzt,

(ii) den Katalysator desaktiviert und

(iii) gegebenenfalls nicht umgesetztes Isocyanat entfernt.

**6.** Verfahren nach Anspruch 5, **dadurch gekennzeichnet, daß** man die Umsetzung so lange durchführt, bis das erhaltene Umsetzungsprodukt nach der Entfernung von noch gegebenenfalls vorhandenem nicht umgesetzten Isophorondiisocyanat oder Hexamethylendiisocyanat eine Viskosität von 100 bis 10000 mPas, gemessen nach ISO 3219, Anhang B aufweist.

**7.** Zwei-Komponenten-Beschichtungsmittel, enthaltend eine Verbindung, die mit Polyisocyanat reagierende Gruppen trägt (A-Komponente), sowie eine Mischung nach Anspruch 1 (I) (B-Komponente).

**8.** Verfahren zum Beschichten von Gegenständen, **dadurch gekennzeichnet, daß** man

- eine Beschichtungszusammensetzung nach Anspruch 7 durch Vermischung der Komponenten (A) und (B) herstellt und

- die Beschichtungszusammensetzung innerhalb von 12 h nach deren Herstellung auf einen Gegenstand flächig aufträgt.

**9.** Beschichtete Gegenstände hergestellt nach Anspruch 8.

## Claims

**1.** A mixture comprising

diisocyanates of the formula (I)

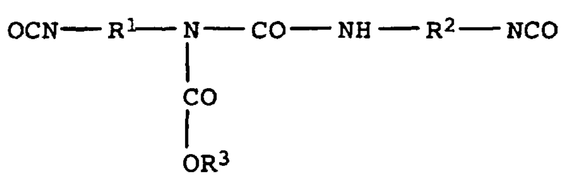

(I)

in which the radicals have the following meanings:

$R^1$, $R^2$: both radicals a radical of the formula (II)

$$-CH_2-CH_2-CH_2-CH_2-CH_2-CH_2-$$

(diisocyanates Ia) (II)

a radical of the formula (II) and the other radical a radical of the formula (III)

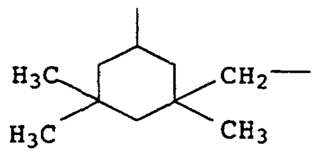

(III)

(diisocyanates Ib)

$R^3$: - a 5- or 6-membered cycloalkyl radical in which up to 3 hydrogen atoms may be substituted by $C_1$-$C_4$-alkyl radicals and one or two methylene units may be substituted by an oxygen atom and/or a tertiary nitrogen atom which additionally carries a $C_1$-$C_4$-alkyl radical, or
- a $C_1$-$C_4$-alkyl radical in which one hydrogen atom is substituted by a 5- or 6-membered cycloalkyl radical in which up to 3 hydrogen atoms may be substituted by $C_1$-$C_4$-alkyl radicals and one or two methylene units may be substituted by an oxygen atom and/or a tertiary nitrogen atom which additionally carries a $C_1$-$C_4$-alkyl radical; a pyrrolidone radical or a morpholine radical, where in the case of the two last-mentioned radicals the nitrogen atom is attached to the alkyl radical,
-urethanes of the formula (IV)

$$OCN\text{-}R^1\text{-}NH\text{-}CO\text{-}OR^3 \qquad (IV)$$

in which the radicals $R^1$ and $R^3$ may have the following meanings:

$R^1$: a radical of the formula (II) or (III)

$R^3$: the meaning indicated in claim 1;

- diisocyanates of the formula (V)

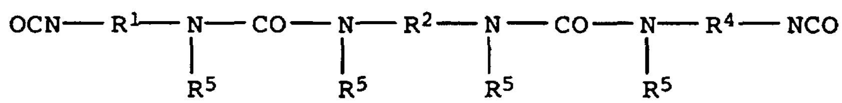

(V)

in which the radicals $R^1$, $R^2$, $R^4$ and $R^5$ may have the following meanings:

$R^1$, $R^2$, $R^4$: the meaning indicated for $R^1$ in formula (I),

$R^5$: 2 of the total of 4 radicals are hydrogen and the other two radicals are a radical of the formula (VI)

$$\text{-}CO\text{-}O\text{-}R^3 \qquad (VI)$$

with the radicals $R^5$ having the same meaning being separated by the unit $R^2$; and
- isocyanurates composed of 3 molecules selected from the group consisting of isophorone diisocyanate and hexamethylene diisocyanate (monoisocyanurates VII),

where the weight ratio of diisocyanates (I) to monoisocyanurates (VII) is from 10:1 to 1:10.

**2.** A diisocyanate as claimed in claim 1, in which the radical $R^3$ is derived from an alcohol selected from the group consisting of cyclohexanol, cyclohexanemethanol, cyclopentanol, cyclopentanemethanol, 3,3,5-trimethylcyclohexanol, menthol, norborneol, N-methyl-4-hydroxypiperidine, 4-(2-hydroxyethyl)-morpholine and 4-(2-hydroxyethyl)pyrrolidone.

**3.** A mixture as claimed in claim 1 or 2, where the proportion of isophorone diisocyanate or hexamethylene diisocyanate is less than 0.5% by weight.

**4.** A mixture as claimed in any of claims 1 to 3, where the sum of the proportions of the diisocyanates (Ia), (Ib), (Ic), (V), the urethane (IV) and the isocyanurate (VII) is from 10 to 100% by weight.

**5.** A process for preparing a mixture as claimed in any of claims 1 to 4, which comprises reacting

(i) isophorone diisocyanate, hexamethylene diisocyanate or a mixture of these isocyanates in the presence of a catalyst with a

- 5- or 6-membered cycloaliphatic alcohol in which up to 3 hydrogen atoms attached to one carbon atom may be substituted by $C_1$-$C_4$-alkyl radicals and one or two methylene units may be substituted by an oxygen atom and/or a tertiary nitrogen atom which additionally carries a $C_1$-$C_4$-alkyl radical, or

- $C_1$-$C_4$-alkyl alcohol in which one hydrogen atom attached to a carbon atom is substituted by a 5- or 6-membered cycloalkyl radical in which up to 3 hydrogen atoms may be substituted by $C_1$-$C_4$-alkyl radicals and one or two methylene units may be substituted by an oxygen atom and/or a tertiary nitrogen atom which additionally carries a $C_1$-$C_4$-alkyl radical; a pyrrolidone radical or morpholine radical, where the two last-mentioned radicals are attached to the alkyl radical of the alcohol by the nitrogen atom;

the molar ratio of said isocyanates to said monoalcohol being from 1.5:1 to 20:1,

(ii) deactivating the catalyst and

(iii) removing any unreacted isocyanate.

**6.** A process as claimed in claim 5, wherein the reaction is continued until the resulting reaction product after removing any unreacted isophorone diisocyanate or hexamethylene diisocyanate still present has a viscosity of from 100 to 10,000 mPas measured in accordance with ISO 3219, Annex B.

**7.** A two-component coating composition comprising a compound which carries polyisocyanate-reactive groups (component A) and a mixture as claimed in claim 1 (I) (component B).

**8.** A method of coating articles which comprises

- preparing a coating composition as claimed in claim 7 by mixing components (A) and (B) and

- applying the coating composition in sheetlike manner to an article within 12 h of the preparation of said composition.

**9.** A coated article produced as claimed in claim 8.

**Revendications**

**1.** Mélanges contenant

- des diisocyanates de la formule (I),

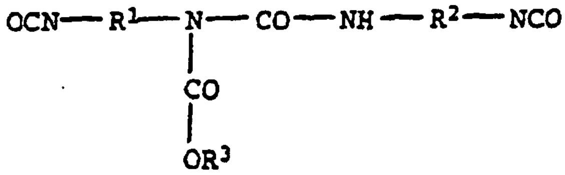

(I)

dans laquelle les restes ont les significations suivantes:

$R^1$, $R^2$: les deux restes sont un reste de la formule (II)

$$-CH_2-CH_2-CH_2-CH_2-CH_2-CH_2-$$

(diisocyanate Ia) (II)

un reste de la formule (II) et l'autre reste est un reste de la formule (III)

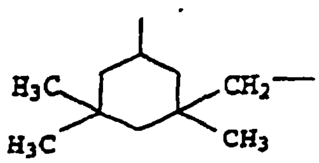

(III)

(diisocyanate Ib)

$R^3$ est un radical cycloalkyle pentagonal ou hexagonal, dans lequel jusqu'à 3 atomes d'hydrogène peuvent être substitués par des radicaux alkyle en $C_1$ à $C_4$, et une ou deux unités méthylène par un atome d'oxygène et/ou un atome d'azote tertiaire, portant en outre un radical alkyle en $C_1$ à $C_4$, ou bien
un radical alkyle en $C_1$ à $C_4$, dans lequel un atome d'hydrogène est substitué par un radical cycloalkyle pentagonal ou hexagonal, dans lequel jusqu'à 3 atomes d'hydrogène peuvent être substitués par des radicaux alkyle en $C_1$ à $C_4$, ou deux unités méthylène par un atome d'oxygène et/ou un atome d'azote tertiaire, portant en outre un radical alkyle en $C_1$ à $C_4$, ou un radical pyrrolidone ou morpholine où, dans ces deux derniers radicaux, l'atome de N est lié au radical alkyle.

des uréthannes de la formule (IV)

$$OCN-R^1-NH-CO-OR^3 \quad (IV)$$

dans laquelle les restes $R^1$ et $R^3$ ont les significations suivantes:

$R^1$ est un reste de formule (II) ou (III)

$R^3$ a la signification indiquée dans la revendication 1,

- des diisocyanates de la formule (V)

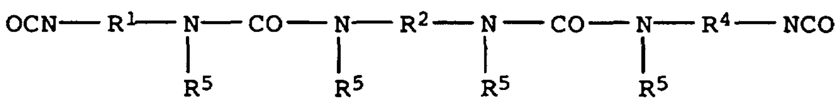

(V)

dans laquelle les restes $R^1$, $R^2$, $R^4$ et $R^5$ peuvent avoir les significations suivantes:

$R^1$, $R^2$, $R^4$ ont les significations indiquées pour la formule (I),
$R^5$ 2 des 4 des 4 restes au total représentent de l'hydrogène, et les deux autres restes représentent un reste de la formule VI

$$-CO-O-R^3 \quad (VI)$$

les restes $R^5$ de même signification étant séparés par l'unité $R^2$, et

- des isocyanurates constitués de 3 molécules choisies dans le groupe formé par le diisocyanate d'isophorone ou le diisocyanate d'hexaméthylène (monoisocyanurates VII),

le rapport pondéral de diisocyanates (I) aux monoisocyanurates (VII) étant de 10:1 à 1:10.

**2.** Diisocyanates selon la revendication 1, dans lesquels le reste $R^3$ est dérivé d'un alcool, choisi dans le groupe formé par le cyclohexanol, le cyclohexane-méthanol, le cyclopentanol, le cyclopentane-méthanol, le 3,3,5-triméthylcyclohexanol, le menthol, le norbornéol, la N-méthyl-4-hydroxypipéridine, la 4-(2-hydroxyéthyl)-morpholine ou la 4-(2-hydroxyéthyl)pyrrolidone.

**3.** Mélanges selon la revendication 1 ou 2, où la fraction diisocyanate d'isophorone ou de diisocyanate d'hexaméthylène est inférieure à 0,5% en poids.

**4.** Mélanges selon les revendications 1 à 3, où la somme des fractions des diisocyanates (Ia), (Ib), (Ic), (V), de l'uréthanne (IV) ainsi que de l'isocyanurate (VII) totalise de 10 à 100% en poids.

**5.** Procédé de préparation de mélanges selon les revendications 3 à 6, **caractérisé en ce que** l'on fait réagir:

(i) du diisocyanate d'isophorone, du diisocyanate d'hexaméthylène ou un mélange de ces isocyanates avec

- un alcool aliphatique pentagonal ou hexagonal, dans lequel jusqu'à 3 atomes d'hydrogène liés à un atome de C peuvent être substitués par des radicaux alkyle en $C_1$ à $C_4$ et une ou deux unités méthylène par un atome d'oxygène et/ou un atome d'azote tertiaire, portant en outre un radical alkyle en $C_1$ à $C_4$, ou bien
- un alkylalcool en $C_1$ à $C_4$, dans lequel un atome d'hydrogène lié à un atome de C est substitué par un radical cycloalkyle pentagonal ou hexagonal, dans lequel jusqu'à 3 atomes d'hydrogène peuvent être substitués par des radicaux alkyle en $C_1$ à $C_4$, et une ou deux unités méthylène par un atome d'oxygène et/ou un atome d'azote tertiaire, portant en outre un radical alkyle en $C_1$ à $C_4$, ou un radical pyrrolidone ou morpholine où ces deux derniers radicaux sont liés au radical alkyle via l'atome de N,

le rapport molaire des isocyanates cités au monoalcool cité étant de 1,5/1 à 20:1, en présence d'un catalyseur,
(ii) on désactive le catalyseur et
(iii) on élimine éventuellement l'isocyanate n'ayant pas réagi.

**6.** Procédé selon la revendication 5, **caractérisé en ce que** l'on poursuit la réaction jusqu'à ce que le produit de réaction obtenu, après élimination du diisocyanate d'isophorone ou du diisocyanate d'hexaméthylène encore éventuellement présent, présente une viscosité de 100 à 10000 mPas, mesurée selon ISO 3219, Annexe B.

**7.** Agent de revêtement à deux composants, contenant un composé portant des groupes réagissant avec du polyisocyanate (composant A) ainsi qu'un mélange selon la revendication 1 (I) (composant B).

**8.** Procédé de revêtement d'objets, **caractérisé en ce que**:

- on prépare une composition de revêtement selon la revendication 7 par mélange des composants (A) et (B) et
- on applique la composition de revêtement en nappe sur l'objet dans les 12 heures qui suivent sa préparation.

**9.** Objets revêtus préparés selon la revendication 8.